# EUROPEAN PATENT SPECIFICATION

(11) **EP 1 996 703 B1**
(45) Date of publication and mention of the grant of the patent: **02.09.2015**
(21) Application number: 07713916.0
(22) Date of filing: 01.02.2007
(51) Int. Cl.: C12N 1/04, C12N 9/10, C12N 15/81, C12N 15/54, C12G 3/02

(54) **GENE ENCODING GLYCOGEN BRANCHING ENZYME AND USE THEREOF**
FÜR DAS GLYKOGEN-VERZWEIGUNGSENZYM CODIERENDES GEN UND VERWENDUNG DAVON
GÈNE CODANT POUR UNE ENZYME DE RAMIFICATION DE GLYCOGÈNE

(30) Priority: 28.02.2006 JP 2006054196
(43) Date of publication of application: 03.12.2008
(73) Proprietor: Suntory Holdings Limited, Kita-ku, Osaka-shi, Osaka 530-8203 (JP)
(72) Inventor: NAKAO, Yoshihiro, Mishima-gun, Osaka 6188503 (JP); KODAMA, Yukiko, Mishima-gun, Osaka 6188503 (JP); SHIMONAGA, Tomoko c/o Suntory Limited, Mishima-gun, Osaka 6188503 (JP)
(74) Representative: Vossius & Partner Patentanwälte Rechtsanwälte mbB
(86) International application number: PCT/JP2007/052168
(87) International publication number: WO 2007/099750

(56) References cited:
- GB-A- 2 360 521
- US-A1- 2004 265 862
- THON V J ET AL: "COORDINATE REGULATION OF GLYCOGEN METABOLISM IN THE YEAST SACCHAROMYCES-CEREVISIAE INDUCTION OF GLYCOGEN BRANCHING ENZYME" JOURNAL OF BIOLOGICAL CHEMISTRY, vol. 267, no. 21, 1992, pages 15224-15228, XP002429194 ISSN: 0021-9258
- DATABASE EMBL [Online] 4 February 1992 (1992-02-04), "Saccharomyces cerevisiae 1,4-glucan-6-(1,4-glucano)-transferase (GLC3) gene, complete cds." XP002429199 retrieved from EBI accession no. EMBL:M76739 Database accession no. M76739
- DATABASE UniProt [Online] 1 October 1993 (1993-10-01), "1,4-alpha-glucan branching enzyme (EC 2.4.1.18) (Glycogen branching enzyme)." XP002429200 retrieved from EBI accession no. UNIPROT:P32775 Database accession no. P32775
- ROWEN D W ET AL: "GLC3 AND GHA1 OF SACCHAROMYCES-CEREVISIAE ARE ALLELIC AND ENCODE THE GLYCOGEN BRANCHING ENZYME" MOLECULAR AND CELLULAR BIOLOGY, vol. 12, no. 1, 1992, pages 22-29, XP002429195 ISSN: 0270-7306
- WILSON W A ET AL: "Increased glycogen storage in yeast results in less branched glycogen" BIOCHEMICAL AND BIOPHYSICAL RESEARCH COMMUNICATIONS, ACADEMIC PRESS INC. ORLANDO, FL, US, vol. 320, no. 2, 23 July 2004 (2004-07-23), pages 416-423, XP004516646 ISSN: 0006-291X
- THON VICKI J ET AL: "Isolation of human glycogen branching enzyme cDNAs by screening complementation in yeast" JOURNAL OF BIOLOGICAL CHEMISTRY, THE AMERICAN SOCIETY OF BIOLOGICAL CHEMISTS, INC.,, US, vol. 268, no. 10, 1993, pages 7509-7513, XP002144326 ISSN: 0021-9258
- SASANGKA P ET AL: "Structural features of the glycogen branching enzyme encoding genes from aspergilli" MICROBIOLOGICAL RESEARCH, FISCHER, JENA,, DE, vol. 157, no. 4, 2002, pages 337-344, XP004956576 ISSN: 0944-5013
- KOSTAL VLADIMIR ET AL: "Low-temperature storage and cold hardiness in two populations of the predatory midge Aphidoletes aphidimyza, differing in diapause intensity" PHYSIOLOGICAL ENTOMOLOGY, vol. 26, no. 4, December 2001 (2001-12), pages 320-328, XP002429196 ISSN: 0307-6962

### SEQUENCE LISTING

<110> Suntory Limited
<120> Gene encoding glycogen branching enzyme and use thereof
<130> PCT06-0143
<150> JP2006-54196
   <151> 2006-02-28
<160> 4
<170> PatentIn version 3.3
<210> 1
   <211> 2115
   <212> DNA
   <213> Saccharomyces sp.
<400> 1
   atgtacaacg ttcctgataa tgtcaaaggt gcagtcgaat tcgatccgtg gctgaaaccc    60
   tttgcggatg tgctatccga gagaagatac ctggccgaca agtggtctta cgatatcact   120
   catgccacgc ccgatggttc gtaccagtcg ctgagtaaat tctctagaga ttcatataag   180
   tcgtatgggt tgcatgccaa cccagatact aaggatatta cctacaaaga atgggcaccc   240
   aatgctcaac gtgcatattt agtgggggag tttaacaact ggaatactac atctcatgag   300
   ctcaaggaca aggacgagtt cggaaatttc accatcacta tccctcccct atcgaatggt   360
   gactttgcca ttcctcatga ctctaagatt aaggtgatgt ttgttttgcc ggatggttct   420
   gagatcttcc gtttacctgc atggattaca agagccactc aaccttctaa ggaaacttcc   480
   aaacaattcg gacctgttta tgaaggaaga ttttggaacc cacaaacctc ttacaatttt   540
   gttaatccaa ggccaaagtt taacggatct gtagactctt taagaatcta cgaagcgcat   600
   gtaggtattt caagtcctga gccaaaggtc actacatata aggaatttac tgaaaaggtt   660
   ttgcccagga ttaaatacct cggttatgat gccattcaat tgatggctat tatggaacat   720
   gcctactatg catcatttgg ttaccaagtg actaatttct ttgcggcaag ctctcgtttt   780
   ggtacccctg aagagttgaa agagctggtc gataccgcac atggaatggg tatcatagtc   840
   ttattggacg ttgttcatag tcacgcttct aaaaatgtag aagatgggct gaatatgttc   900
   gatggctccg atcatcaata tttccattct gtaagttccg gtagaggtga acatccatta   960
   tgggattctc gtctttttaa ctacggtaaa tttgaagtac aacgatttct attggcaaat  1020
   ctggcttttt atgtcgacgt ttatcagttt gatggtttta gattcgacgg tgtcacttcc  1080
   atgctatatg ttcatcatgg tgttggtgaa ggcggttcct ttagtggtga ctacaacgaa  1140
   tacctttctc gtgatagatc ttctgtcgac catgaggctt tagcttattt gatgttagcc  1200
   aacgatttag ttcacgaatt actgcctcag tcggctgtca ctattgcgga agatgtgtct  1260
   ggttacccaa ctctctgtct accccgttcc attggtggta gtggtttcga ctacagattg  1320
   gccatggctt tacccgatat gtggattaaa ttaatcaagg agaaagcaga cgatgaatgg  1380
   gaaatgggcg gtatcgttta cactttgact aatagacgtc atggtgaaaa agtcgttgcc  1440
   tattgtgaat ctcacgatca agctttggtt ggtgacaaga ccttagcatt ttggttgatg  1500
   gatgcctcaa tgtataccga tatgactgta ttgaaggaac ctaccattat cattgatcgt  1560
   ggtattgccc tacacaagat gattaggtta attactcatt cattgggtgg tgaagcttat  1620
   ttgaacttcg aaggtaacga gtttggccac ccagaatggt tggatttccc caatgctaac  1680
   aatggcgaca gttataagta cgctcggaga caatttaatt tggcagatga tcctttgtta  1740
   cgctaccaga atttgaatga atttgataga tcaatgcagt tatgtgagaa aaaacataag  1800
   tggttgaaca cagaacaagc atatgtctca ttaaagaatg aaaaagacaa aatgattgtg  1860
   tttgaaagaa ataatttact atttattttc aactttcatc ccacagacag ctacagtgat  1920
   tatagagttg gtgttgaaaa agctggtact tatcacatcg tattaaactc ggatcgtgct  1980
   gaatttggcg gacataacag aatcaacgaa acttccgaat tctttaccac tgacttagaa  2040
   tggaataaca ggagaaattt tattcaggta tacattccta gcagagtttg tttggttctt  2100
   gctttgaaag aatga                                                   2115
<210> 2
   <211> 704
   <212> PRT
   <213> Saccharomyces sp.
<400> 2
   Met Tyr Asn Val Pro Asp Asn Val Lys Gly Ala Val Glu Phe Asp Pro
   1               5                   10                  15
   Trp Leu Lys Pro Phe Ala Asp Val Leu Ser Glu Arg Arg Tyr Leu Ala
               20                  25                  30
   Asp Lys Trp Ser Tyr Asp Ile Thr His Ala Thr Pro Asp Gly Ser Tyr
           35                  40                  45
   Gln Ser Leu Ser Lys Phe Ser Arg Asp Ser Tyr Lys Ser Tyr Gly Leu
       50                  55                  60
   His Ala Asn Pro Asp Thr Lys Asp Ile Thr Tyr Lys Glu Trp Ala Pro
   65                  70                  75                  80
   Asn Ala Gln Arg Ala Tyr Leu Val Gly Glu Phe Asn Asn Trp Asn Thr
                   85                  90                  95
   Thr Ser His Glu Leu Lys Asp Lys Asp Glu Phe Gly Asn Phe Thr Ile
                   100                 105                 110
   Thr Ile Pro Pro Leu Ser Asn Gly Asp Phe Ala Ile Pro His Asp Ser
               115                 120                 125
   Lys Ile Lys Val Met Phe Val Leu Pro Asp Gly Ser Glu Ile Phe Arg
           130                 135                 140
   Leu Pro Ala Trp Ile Thr Arg Ala Thr Gln Pro Ser Lys Glu Thr Ser
   145                 150                 155                 160
   Lys Gln Phe Gly Pro Val Tyr Glu Gly Arg Phe Trp Asn Pro Gln Thr
                   165                 170                 175
   Ser Tyr Asn Phe Val Asn Pro Arg Pro Lys Phe Asn Gly Ser Val Asp
               180                 185                 190
   Ser Leu Arg Ile Tyr Glu Ala His Val Gly Ile Ser Ser Pro Glu Pro
           195                 200                 205
   Lys Val Thr Thr Tyr Lys Glu Phe Thr Glu Lys Val Leu Pro Arg Ile
       210                 215                 220
   Lys Tyr Leu Gly Tyr Asp Ala Ile Gln Leu Met Ala Ile Met Glu His
   225                 230                 235                 240
   Ala Tyr Tyr Ala Ser Phe Gly Tyr Gln Val Thr Asn Phe Phe Ala Ala
                   245                 250                 255
   Ser Ser Arg Phe Gly Thr Pro Glu Glu Leu Lys Glu Leu Val Asp Thr
               260                 265                 270
   Ala His Gly Met Gly Ile Ile Val Leu Leu Asp Val Val His Ser His
           275                 280                 285
   Ala Ser Lys Asn Val Glu Asp Gly Leu Asn Met Phe Asp Gly Ser Asp
       290                 295                 300
   His Gln Tyr Phe His Ser Val Ser Ser Gly Arg Gly Glu His Pro Leu
   305                 310                 315                 320
   Trp Asp Ser Arg Leu Phe Asn Tyr Gly Lys Phe Glu Val Gln Arg Phe
                   325                 330                 335
   Leu Leu Ala Asn Leu Ala Phe Tyr Val Asp Val Tyr Gln Phe Asp Gly
               340                 345                 350
   Phe Arg Phe Asp Gly Val Thr Ser Met Leu Tyr Val His His Gly Val
           355                 360                 365
   Gly Glu Gly Gly Ser Phe Ser Gly Asp Tyr Asn Glu Tyr Leu Ser Arg
       370                 375                 380
   Asp Arg Ser Ser Val Asp His Glu Ala Leu Ala Tyr Leu Met Leu Ala
   385                 390                 395                 400
   Asn Asp Leu Val His Glu Leu Leu Pro Gln Ser Ala Val Thr Ile Ala
                   405                 410                 415
   Glu Asp Val Ser Gly Tyr Pro Thr Leu Cys Leu Pro Arg Ser Ile Gly
               420                 425                 430
   Gly Ser Gly Phe Asp Tyr Arg Leu Ala Met Ala Leu Pro Asp Met Trp
           435                 440                 445
   Ile Lys Leu Ile Lys Glu Lys Ala Asp Asp Glu Trp Glu Met Gly Gly
       450                 455                 460
   Ile Val Tyr Thr Leu Thr Asn Arg Arg His Gly Glu Lys Val Val Ala
   465                 470                 475                 480
   Tyr Cys Glu Ser His Asp Gln Ala Leu Val Gly Asp Lys Thr Leu Ala
                   485                 490                 495
   Phe Trp Leu Met Asp Ala Ser Met Tyr Thr Asp Met Thr Val Leu Lys
               500                 505                 510
   Glu Pro Thr Ile Ile Ile Asp Arg Gly Ile Ala Leu His Lys Met Ile
           515                 520                 525
   Arg Leu Ile Thr His Ser Leu Gly Gly Glu Ala Tyr Leu Asn Phe Glu
       530                 535                 540
   Gly Asn Glu Phe Gly His Pro Glu Trp Leu Asp Phe Pro Asn Ala Asn
   545                 550                 555                 560
   Asn Gly Asp Ser Tyr Lys Tyr Ala Arg Arg Gln Phe Asn Leu Ala Asp
                   565                 570                 575
   Asp Pro Leu Leu Arg Tyr Gln Asn Leu Asn Glu Phe Asp Arg Ser Met
               580                 585                 590
   Gln Leu Cys Glu Lys Lys His Lys Trp Leu Asn Thr Glu Gln Ala Tyr
           595                 600                 605
   Val Ser Leu Lys Asn Glu Lys Asp Lys Met Ile Val Phe Glu Arg Asn
       610                 615                 620
   Asn Leu Leu Phe Ile Phe Asn Phe His Pro Thr Asp Ser Tyr Ser Asp
   625                 630                 635                 640
   Tyr Arg Val Gly Val Glu Lys Ala Gly Thr Tyr His Ile Val Leu Asn
                   645                 650                 655
   Ser Asp Arg Ala Glu Phe Gly Gly His Asn Arg Ile Asn Glu Thr Ser
               660                 665                 670
   Glu Phe Phe Thr Thr Asp Leu Glu Trp Asn Asn Arg Arg Asn Phe Ile
           675                 680                 685
   Gln Val Tyr Ile Pro Ser Arg Val Cys Leu Val Leu Ala Leu Lys Glu
       690                 695                 700
<210> 3
   <211> 40
   <212> DNA
   <213> Artificial
<220>
   <223> Primer
<400> 3
   gagctcatag cggccatgta caacgttcct gataatgtca 40
<210> 4
   <211> 42
   <212> DNA
   <213> Artificial
<220>
   <223> Primer
<400> 4
   ggatcctatg cggccgcttt atatatgaaa aggaaggaag ca 42

## Description

### TECHNICAL FIELD

The present invention relates to a gene encoding glycogen branching enzyme and use thereof, in particular, a yeast for practical use with superior resistance property to dryness and/or resistance property to low-temperature storage, alcoholic beverages produced with said yeast, and a method for producing said beverages. More particularly, the present invention relates to a yeast, whose resistance property to dryness and/or resistance property to low-temperature storage is enhanced by amplifying expression level of GLC3 gene encoding a protein Glc3p having a glycogen branching enzyme activity in brewer's yeast, especially non-ScGLC3 gene specific to a lager brewing yeast and to a method for producing alcoholic beverages with said yeast, etc. Further, the yeast of the present invention is useful as a baker's yeast or an industrial yeast as well.

### BACKGROUND ART

Beer brewing is characterized by a process recovering yeasts after fermentation and using the recovered yeasts at the subsequent fermentation, which is called "Renjo". The yeasts are stored in the presence of ethanol in a tank whose temperature is kept at approximately 0 to 3°C. When the yeasts die during the storage, not only the next fermentation process is interfered, but also constituents of the yeast cells released by cell lysis may impart unfavorable taste to product. Therefore, it is very important for allowing some variance to design production process and for stable production of quality products to use yeasts with superior resistant property to low temperature storage.

"Renjo" may be terminated at a certain times of fermentation is carried out. The number of times of "Renjo" may vary according to fermentation conditions or properties of yeasts used in the process. A process to develop yeasts for fermentation freshly is called propagation. Yeasts are subcultured several times enlarging scales of culture successively during the propagation process. Because propagation process requires from several days to several weeks, it brings great advantages in production efficiency if term of the process is shortened or yeast cells which are large-scale pre-cultured are able to be stored stably for extended period of time at low temperature or under dry condition.

Concerning a method for producing dry yeast maintaining high viable cell ratio, improvement of drying equipment, or improvement of manufacturing conditions such as temperature or addition of emulsifiers, etc., have been made. For example, L-drying method is not practical to be used at industrial production scale because, though it can maintain extremely high viable cell ratio, but at the same time it takes a lot of time and cost.

Regarding low-temperature resistance of yeast, some experiments designed to improve refrigeration-resistant property mainly of baker's yeast were reported. This is because Saccharomyces cerevisiae, which is a baker's yeast, has poor low-temperature storage property in comparison with brewer's yeast for beer or sake, which can ferment at low temperature. For example, baker's yeasts having refrigeration-resistant property and drying-resistant property were found out mainly by screening methods in Japanese Patent Application Laid-open No. H11-155559 and Japanese Patent Application Laid-open No. 2003-304864. Further, regarding examples utilizing genetic engineering techniques, trehalose highly accumulating strains by disruption of NTH1, which is a trehalase gene, is reported in Japanese Patent Application Laid-open No. H10-117771 and a strain highly accumulating specific amino acids such as arginine by disruption of CAR1, which is an arginase gene, is reported in Japanese Patent Application Laid-open No. 2001-238665. Thon et al. (J. Biol. Chem. 267 (1992), 15224-15228) describes a Saccharomyces cerevisiae glycogen branching enzyme named GLC3 and its expression during growth of S. cerevisiae in culture as well as its involvement in glycogen biosynthesis.

### DISCLOSURE OF INVENTION

Under the above situations, there has been a need to make high-efficiency production of alcoholic beverages or useful materials possible by using a gene encoding a protein responsible for drying and/or low-temperature storage-resistant property of brewery yeast and said protein.

The present inventors made extensive studies to solve the above problems and as a result, succeeded in identifying and isolating a gene encoding glycogen branching enzyme from beer yeast. Moreover, the present inventors produced transformed yeast in which the obtained gene was expressed to verify that drying-resistant property and/or low-temperature storage-resistant property can be actually improved, thereby completing the present invention.

Thus, the present invention relates to a gene encoding a glycogen branching enzyme of brewery yeast as described below, to a protein encoded by said gene, to a yeast into which a vector containing said gene has been introduced and to a method for enhancing drying-resistant property of yeast using a yeast in which the expression of said gene is controlled. More specifically, the present invention provides the following polynucleotides, a vector comprising said polynucleotide, a transformed yeast introduced with said vector and a method for producing alcoholic beverages by using said transformed yeast:
(1) A polynucleotide selected from the group consisting of
   (a) a polynucleotide comprising a polynucleotide consisting of the nucleotide sequence of SEQ ID NO: 1;
   (b) a polynucleotide comprising a polynucleotide encoding a protein consisting of the amino acid sequence of SEQ ID NO: 2;
   (c) a polynucleotide comprising a polynucleotide encoding a protein consisting of the amino acid sequence of SEQ ID NO: 2 in which 1 to 40 amino acids thereof are deleted, substitutes, inserted and/or added, and having a glycogen branching enzyme activity; and
   (d) a polynucleotide comprising a polynucleotide encoding a protein having an amino acid sequence having 94% or higher identity with the amino acid sequence of SEQ ID NO: 2, and said protein having a glycogen branching enzyme activity.
(2) The polynucleotide according to (1) above selected from the group consisting of:
   (g) a polynucleotide comprising a polynucleotide encoding a protein consisting of the amino acid sequence of SEQ ID NO: 2, or encoding the amino acid sequence of SEQ ID NO: 2 in which 1 to 5 amino acids thereof are deleted, substituted, inserted, and/or added, and wherein said protein has a glycogen branching enzyme activity; and
   (h) a polynucleotide comprising a polynucleotides encoding a protein having 99% or higher identity with the amino acid sequence of SEQ ID NO: 2, and having a glycogen branching enzyme activity.
(3) The polynucleotide according to (1) above comprising a polynucleotides consisting of the nucleotide sequence of SEQ ID NO: 1.
(4) The polynucleotide according to (1) above comprising a polynucleotide encoding a protein consisting of the amino acid sequence of SEQ ID NO: 2.
(5) The polynucleotide according to any one of (1) to (4) above, wherein the polynucleotide is DNA.
(6) A protein encoded by the polynucleotide according to any one of (1) to (5) above.
(7) A vector containing the polynucleotide according to any one of (1) to (5) above.
   The vector of (7) above may comprise an expression cassette comprising the following components:
   (x) a promoter that can be transcribed in a yeast cell;
   (y) any of the polynucleotides described in (1) to (5) above linked to the promoter in a sense or antisense direction; and
   (z) a signal that can function in a yeast with respect to transcription germination and polyadenylation of a RNA molecule.

   The vector of (7) above may comprise an expression cassette comprising the following components:
   (x) a promoter that can be transcribed in a yeast cell;
   (y) any of the polynucleotides described in (1) to (5) above linked to the promoter in a sense direction; and
   (z) a signal that can function in a yeast with respect to transcription termination and polyadenylation of a RNA molecule.
(8) A yeast into which the vector according to (7) above has been introduced.
   The yeast (yeast for practical use) according to (8) above preferably shows an increase in drying-resistant property. The "yeast for practical use" means that a yeast which possesses practical value such as brewer's (brewery) yeast, baker's yeast or industrial yeast, etc.
   The yeast according to (8) above, preferably shows an increase in low-temperature storage-resistant property.
   The drying-resistant property is increased by increasing an expression level of the protein of (6) above.
   The low-temperature storage-resistant property is increased by increasing an expression level of the protein of (6) above.
   The yeast is preferably a brewery yeast.
(9) A method for producing an alcoholic beverage by using the yeast according to (8) above.
(10) The method according to 9 above, wherein the brewed alcoholic beverage is a malt beverage.
(11) The method according to (9) above, wherein the brewed alcoholic beverage is wine.
(12) Use of a polynucleotide of any one of (1) to (5) above or of a protein of (6) above or of a vector of (7) above for increasing the drying-resistance in yeast cells.
(13) A method for increasing the drying-resistance in a yeast cell comprising the step of transforming a yeast cell with a polynucleotide of any one of (1) to (5) above or with a vector of (7) above and expressing the glycogen branching enzyme encoded by the polynucleotide of any one of (1) to (5) in the transformed yeast cell.

The transformed yeast of the present invention is able to keep high viable cell count during dry storage or low-temperature storage. Therefore, when it is used for brewing and so on, painfulness of conserving yeast can be eliminated. Further, it is expected to contribute to quality stabilization. Moreover, dry yeast is suitable for long-storage, and it is very advantageous to distribution or transportation due to its reduced weight. It is also useful as microorganisms for industrial application such as industrial alcohol production or production of useful proteins. The yeast of the present invention also useful as an industrial yeast as well.

### BRIEF DESCRIPTION OF DRAWINGS

Figure 1 shows the cell growth with time upon beer fermentation test. The horizontal axis represents fermentation time while the vertical axis represents optical density at 660 nm (OD660).
Figure 2 shows the extract (sugar) consumption with time upon beer fermentation test. The horizontal axis represents fermentation time while the vertical axis represents apparent extract concentration (w/w%).
Figure 3 shows the expression profile of non-ScGLC3 gene in yeasts upon beer fermentation test. The horizontal axis represents fermentation time while the vertical axis represents the intensity of detected signal.
Figure 4 shows the result of drying-resistant property test of parent strain and non-ScGLC3 highly expressed strain.

### BEST MODES FOR CARRYING OUT THE INVENTION

The present inventors isolated and identified non-ScGLC3 gene encoding a glycogen branching enzyme of brewery yeast based on the lager brewing yeast genome information mapped according to the method disclosed in Japanese Patent Application Laid-Open No. 2004-283169. The nucleotide sequence of the gene is represented by SEQ ID NO: 1. Further, an amino acid sequence of a protein encoded by the gene is represented by SEQ ID NO: 2.

### 1. Polynucleotide of the invention

First of all, the present invention provides (a) a polynucleotide comprising a polynucleotide of the nucleotide sequence of SEQ ID NO: 1; and (b) a polynucleotide comprising a polynucleotide encoding a protein of the amino acid sequence of SEQ ID NO: 2. The polynucleotide can be DNA or RNA

The target polynucleotide of the present invention is not limited to the polynucleotide encoding a protein having a glycogen branching enzyme activity described above and may include other polynucleotides encoding proteins having equivalent functions to said protein as described below. Proteins with equivalent functions include, for example, (c) a protein of an amino acid sequence of SEQ ID NO: 2 with 1 to 40 amino acids thereof being deleted, substituted, inserted and/or added and having a glycogen branching enzyme activity.

Such proteins include a protein consisting of an amino acid sequence of SEQ ID NO: 2 with 1 to 40, 1 to 39, 1 to 38, 1 to 37, 1 to 36, 1 to 35, 1 to 34, 1 to 33, 1 to 32, to 31, 1 to 30, 1 to 29, 1 to 28, 1 to 27, 1 to 26, 1 to 25, 1 to 24, to 23, 1 to 22, 1 to 21, 1 to 20, 1 to 19, 1 to 18, 1 to 17, 1 to 16, 1 to 15, 1 to 14, 1 to 13, 1 to 12, 1 to 11, 1 to 10, 1 to 9, 1 to 8, 1 to 7, 1 to 6 (1 to several amino acids), 1 to 5, 1 to 4, 1 to 3, 1 to 2, or 1 amino acid residues thereof being deleted, substituted, inserted and/or added and having a glycogen branching enzyme activity. In general, the number of deletions, substitutions, insertions, and/or additions is preferably smaller. In addition, such proteins include (d) a protein having an amino acid sequence with 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO: 2, and having a glycogen branching enzyme activity. In general, the percentage identity is preferably higher.

Glycogen branching enzyme activity may be measured, for example, by a method described in Mol. Cell. Biol., 1992 Jan; 12(1): 22-9.

Identity between amino acid sequences or nucleotide sequences may be determined using algorithm BLAST by Karlin and Altschul (Proc. Natl. Acad. Sci. USA, 87: 2264-2268, 1990; Proc. Natl. Acad. Sci. USA, 90:5873, 1993). Programs called BLASTN and BLASTX based on BLAST algorithm have been developed (Altschul SF et aL, J. Mol. Biol. 215: 403, 1990). When a nucleotide sequence is sequenced using BLASTN, the parameters are, for example, score = 100 and word length = 12. When an amino acid sequence is sequenced using BLASTX, the parameters are, for example, score = 50 and word length = 3. When BLAST and Gapped BLAST programs are used, default parameters for each of the programs are employed.

### 2. Protein of the present invention

The present invention also provides proteins encoded by any of the polynucleotides (a) to (d), (g) or (h) above. A preferred protein of the present invention comprises an amino acid sequence of SEQ ID NO: 2 with 1 to 40 amino acids thereof being deleted, substituted, inserted and/or added, and having a glycogen branching enzyme activity.

Such protein includes those having an amino acid sequence of SEQ ID NO: 2 with amino acid residues thereof of the number mentioned above being deleted, substituted, inserted and/or added and having a glycogen branching enzyme activity. In addition, such protein includes those having homology as described above with the amino acid sequence of SEQ ID NO: 2 and having a glycogen branching enzyme activity.

Such proteins maybe obtained by employing site-directed mutation described, for example, in MOLECULAR CLONING 3rd Ed., CURRENT PROTOCOLS IN MOLECULAR BIOLOGY, Nuc. Acids. Res., 10: 6487 (1982), Proc. Natl. Acad. Sci. USA 79: 6409 (1982), Gene 34: 315 (1985), Nuc. Acids. Res., 13: 4431 (1985), Proc. Natl. Acad. Sci. USA 82: 488 (1985).

Deletion, substitution, insertion and/or addition of 1 to 40 amino acid residues in an amino acid sequence of the protein of the invention means that 1 to 40 amino acid residues are deleted, substituted, inserted and/or added at any one or more positions in the same amino add sequence. Two or more types of deletion, substitution, insertion and/or addition may occur concurrently.

Hereinafter, examples of mutually substitutable amino acid residues are enumerated. Amino acid residues in the same group are mutually substitutable. The groups are provided below.

Group A: leucine, isoleucine, norleucine, valine, norvaline, alanine, 2-aminobutanoic acid, methionine, o-methylserine, t-butylglycine, t-butylalanine, cyclohexylalanine; Group B: asparatic acid, glutamic acid, isoasparatic acid, isoglutamic acid, 2-aminoadipic acid, 2-aminosuberic acid; Group C: asparagine, glutamine; Group D: lysine, arginine, ornithine, 2,4-diaminobutanoic acid, 2,3-diaminopropionic acid; Croup E: proline, 3-hydroxyproline, 4-hydroxyproline; Group F: serine, threonine, homoserine; and Group G: phenylalanine, tyrosine.

The protein of the present invention may also be produced by chemical synthesis methods such as Fmoc method (fluorenylmethyloxycarbonyl method) and tBoc method (t-butyloxycarbonyl method). In addition, peptide synthesizers available from, for example, Advanced ChemTech, PerkinElmer, Pharmacia, Protein Technology Instrument, Synthecell-Vega, PerSeptive, Shimazu Corp. can also be used for chemical synthesis.

### 3. Vector of the invention and yeast transformed with the vector

The present invention then provides a vector comprising the polynucleotide described above. The vector of the present invention is directed to a vector including any of the polynucleotides described in (a) to (d), (g) or (h) above.

Generally, the vector of the present invention comprises an expression cassette including as components (x) a promoter that can transcribe in a yeast cell; (y) a polynucleotide described in any of (a) to (d), (g) or (h) above that is linked to the promoter in sense or antisense direction; and (z) a signal that functions in the yeast with respect to transcription termination and polyadenylation of RNA molecule. Further, in order to highly express the protein of the invention, these polynucleotides are preferably introduced in the sense direction to the promoter to promote expression of the polynucleotide (DNA) described in any of (a) to (d), (g) or (h) above.

A vector introduced in the yeast may be any of a multicopy type (YEp type), a single copy type (YCp type), or a chromosome integration type (YIp type). For example, YEp24 (J. R. Broach et al., EXPERMENTAL MANIPULATION OF GENE EXPRESSION, Academic Press, New York, 83, 1983) is known as a YEp type vector, YCp50 (M. D. Rose et al., Gene 60: 237, 1987) is known as a YCp type vector, and YIp5 (K. Struhl et aL, Proc Natl. Acad. Sci. USA, 76: 1035, 1979) is known as a YIp type vector, all of which are readily available.

Promoters/terminators for adjusting gene expression in yeast may be in any combination as long as they function in the yeast for practical use and they are not influenced by constituents in fermentation broth. For example, a promoter of glyceraldehydes 3-phosphate dehydrogenase gene (TDH3), or a promoter of 3 phosphoglycerate kinase gene (PGK1) may be used. These genes halve previously been cloned, described in detail, for example, in M F. Tuite et al., EMBO J., 1, 603 (1982), and are readily available by known methods.

Since an auxotrophy marker cannot be used as a selective marker upon transformation for a yeast for practical use, for example, a geneticin-resistant gene (G418r), a copper-resistant gene (CUP1) (Marin et al., Proc. Natl. Acad. Sci. USA, 81,337 1984) or a cerulenin-resistant gene (fas2m, PDR4) (Junji Inokoshi et al., Biochemistry, 64, 660, 1992; and Hussain et al., Gene, 101: 149, 1991, respectively) maybe used.

A vector constructed as described above is introduced into a host yeast. Examples of the host yeast include any yeast (yeast for practical use) that can be used for brewing, for example, brewery yeasts for beer, wine and sake, baker's yeast, yeast for producing industrial alcohol or yeast for producing useful proteins and so on. Specifically, yeasts such as genus *Saccharomyces* may be used. According to the present invention, a lager brewing yeast, for example, *Saccharomyces pastorianus* W34/70, etc., *Saccharomyces carlsbergensis* NCYC453 or NCYC456, etc., or *Saccharomyces cerevisiae* NBRC1951, NBRC1952, NBRC1953 or NBRC1954, etc., may be used. In addition, whisky yeasts such as *Saccharomyces cerevisiae* NCYC90, wine yeasts such as wine yeasts #1, 3 and 4 from the Brewing Society of Japan, and sake yeasts such as sake yeast #7 and 9 from the Brewing Society of Japan, baker's yeast such as NBRC0555, NBRC1346 or NBRC2043, etc., may also be used but not limited thereto. In the present invention, lager brewing yeasts such as *Saccharomyces pastorianus* may be used preferably.

A yeast transformation method may be a generally used known method. For example, methods that can be used include but not limited to an electroporation method (Meth Enzym., 194: 182 (1990)), a spheroplast method (Proc. Natl. Acad Sci USA, 75: 1929(1978)), a lithium acetate method (J. Bacteriology, 153: 163 (1983)), and methods described in Proc. Natl. Acad. Sci. USA, 75: 1929 (1978), METHODS IN YEAST GENETICS, 2000 Edition: A Cold Spring Harbor Laboratory Course Manual.

More specifically, a host yeast is cultured in a standard yeast nutrition medium (e.g., YEPD medium (Genetic Engineering. Vol. 1, Plenum Press, New York, 117(1979)), etc.) such that OD600 nm will be 1 to 6. This culture yeast is collected by centrifugation, washed and pre-treated with alkali metal ion, preferably lithium ion at a concentration of about 1 to 2 M. After the cell is left to stand at about 30°C for about 60 minutes, it is left to stand with DNA to be introduced (about 1 to 20 µg) at about 30°C for about another 60 minutes. Polyethyleneglycol, preferably about 4,000 Dalton of polyethyleneglycol, is added to a final concentration of about 20% to 50%. After leaving at about 30°C for about 30 minutes, the cell is heated at about 42°C for about 5 minutes. Preferably, this cell suspension is washed with a standard yeast nutrition medium, added to a predetermined amount of fresh standard yeast nutrition medium and left to stand at about 3 0°C for about 60 minutes. Thereafter, it is seeded to a standard agar medium containing an antibiotic or the like as a selective marker to obtain a transformant.

Other general cloning techniques may be found, for example, in molecular CLONING 3rd Ed., and METHODS IN YEAST GENETICS, A LABORATORY MANUAL (Cold Spring Harbor Laboratory Press, Cold Spring Harbor, NY).

### 4. Method of producing alcoholic beverages according to the present invention and alcoholic beverage produced by the method

A yeast having a superior drying resistant property and/or low-temperature storage-resistant property can be obtained by introducing the vector of the present invention described above to a yeast. Further, a yeast having a superior drying-resistant property and/or low-temperature storage-resistant property can be obtained by selecting a yeast by the yeast assessment method of the present invention described below. The target use of yeasts obtained in the present invention include, for example, but not limited to, brewing alcoholic beverages such as beer, wine, whisky, sake and the like, baking bread, manufacturing useful materials such as industrial alcohol production and production of useful proteins.

In order to produce these products, a known technique can be used except that a yeast for practical use obtained according to the present invention is used in the place of a parent strain. Since starting materials, manufacturing equipment, manufacturing control and the like may be the same as the conventional ones; it can be performed without increasing cost.

### 5. Yeast assessment method of the invention

The present invention also describes a method for assessing a test yeast for its drying-resistant property and/or low-temperature storage-resistant property by using a primer or a probe designed based on a nucleotide sequence of a gene having the nucleotide sequence of SEQ ID NO: 1 and encoding a glycogen branching enzyme. General technique for such assessment method is known and is described in, for example, WO01/040514, Japanese Laid-Open Patent Application No. H8-205900 or the like. This assessment method is described in below.

First, genome of a test yeast is prepared. For this preparation, any known method such as Hereford method or potassium acetate method may be used (e.g., METHODS IN YEAST GENETICS, Cold Spring Harbor Laboratory Press, 130 (1990)). Using a primer or a probe designed based on a nucleotide sequence (preferably, ORF sequence) of the gene encoding a glycogen branching enzyme, the existence of the gene or a sequence specific to the gene is determined in the test yeast genome obtained. The primer or the probe may be designed according to a known technique.

Detection of the gene or the specific sequence may be carried out by employing a known technique. For example, a polynucleotide including part or all of the specific sequence or a polynucleotide including a nucleotide sequence complementary to said nucleotide sequence is used as one primer, while a polynucleotide including part or all of the sequence upstream or downstream from this sequence or a polynucleotide including a nucleotide sequence complementary to said nucleotide sequence, is used as another primer to amplify a nucleic acid of the yeast by a PCR method, thereby determining the existence of amplified products and molecular weight of the amplified products. The number of bases of polynucleotide used for a primer is generally 10 base pairs (bp) or more, and preferably 15 to 25 bp. In general, the number of bases between the primers is suitably 300 to 2000 bp.

The reaction conditions for PCR are not particularly limited but may be, for example, a denaturation temperature of 90 to 95°C, an annealing temperature of 40 to 60°C, an elongation temperature of 60 to 75°C, and the number of cycle of 10 or more. The resulting reaction product may be separated, for example, by electrophoresis using agarose gel to determine the molecular weight of the amplified product. This method allows prediction and assessment of the drying-resistant property and/or low-temperature storage-resistant property of yeast as determined by whether the molecular weight of the amplified product is a size that contains the DNA molecule of the specific part. In addition, by analyzing the nucleotide sequence of the amplified product, the property may be predicted and/or assessed more precisely.

Moreover, it is also conceivable that a test yeast is cultured to measure an expression level of the gene encoding a glycogen branching enzyme having the nucleotide sequence of SEQ ID NO: 1 to assess the test yeast for its drying-resistant, property and/or low-temperature storage-resistant property. Measurement of expression level of the gene encoding a glycogen branching enzyme can be performed by culturing test yeast and then quantifying mRNA or a protein resulting from the gene. The quantification of mRNA or protein may be carried out by employing a known technique. For example, mRNA may be quantified, by Northern hybridization or quantitative RT-PCR, while protein may be quantified, for example, by Western blotting (CURRENT PROTOCOLS IN MOLECULAR BIOLOGY, John Wiley & Sons 1994-2003).

Furthermore, test yeasts are cultured and expression levels of the gene encoding a glycogen branching enzyme having the nucleotide sequence of SEQ ID NO: 1 are measured to select a test yeast with the gene expression level according to the target glycogen-producing ability, thereby a yeast favorable for brewing desired alcoholic beverages can be selected. In addition, a reference yeast and a test yeast may be cultured so as to measure and compare the expression level of the gene in each of the yeasts, thereby a favorable test yeast can be selected. More specifically, for example, a reference yeast and one or more test yeasts are cultured and an expression level of the gene encoding a glycogen branching enzyme having the nucleotide sequence of SEQ ID NO: 1 is measured in each yeast By selecting a test yeast with the gene expressed higher than that in the reference yeast a yeast suitable for brewing desired alcoholic beverages or production of useful materials can be selected.

Alternatively, test yeasts are cultured and a yeast with a high glycogen branching enzyme activity is selected, thereby a yeast suitable for brewing desired alcoholic beverages or production of useful materials can be selected.

In these cases, the test yeasts or the reference yeast may be, for example, a yeast introduced with the vector of the invention, an artificially mutated yeast or a naturally mutated yeast The glycogen branching enzyme activity can be measured by, for example, a method described in Eur J Biochem. 1993 Mar 1: 212(2): 315-23. The mutation treatment may employ any methods including, for example, physical methods such as ultraviolet irradiation and radiation irradiation, and chemical methods associated with treatments with drugs such as EMS (ethylmethane sulphonate) and N-methyl-N-nitrosoguanidine (*see*, *e*.*g*., Yasuji Oshima Ed., BIOCHEMISTRY EXPERIMENTS vol. 39, Yeast Molecular Genetic Experiments, pp. 67-75, JSSP).

In addition, examples of yeasts used as the reference yeast or the test yeasts include any yeasts (yeasts for practical use), for example, brewery yeasts for beer, wine, sake and the like or baker's yeast, yeast for producing industrial alcohol or yeast for producing useful proteins, etc. More specifically yeasts such as genus *Saccharomyces* may be used (*e*.*g*., *S. pastorianus*, *S*. *cerevisiae*, and *S. carlsbergensis*). According to the present invention, a lager brewing yeast, for example, *Saccharomyces pastorianus* W34/70; *Saccharomyces carlsbergensis* NCYC453 or NCYC456; or *Saccharomyces cerevisiae* NBRC1951, NBRC1952, NBRC1953 or NBRC1954, etc., may be used. Further, wine yeasts such as wine yeasts #1, 3 and 4 from the Brewing Society of Japan; and sake yeasts such as sake yeast #7 and 9 from the Brewing Society of Japan, baker's yeast such as NBRC0555, NBRC1346 and NBRC2043, etc., may also be used but not limited thereto. In the present invention, lager brewing yeasts such as *Saccharomyces pastorianus* may preferably be used. The reference yeast and the test yeasts may be selected from the above yeasts in any combination.

### EXAMPLES

Hereinafter, the present invention will be described in more detail with reference to working examples. The present invention, however, is not limited to the examples described below.

### Example 1: Cloning of Gene Encoding Glycogen Branching Enzyme (non-ScGLC3)

A gene encoding a glycogen branching enzyme of lager brewing yeast (non-ScGLC3) (SEQ ID NO: 1) was found as a result of a search utilizing the comparison database described in Japanese Patent Application Laid-Open No. 2004-283169. Based on the acquired nucleotide sequence information, primers non-ScGLC3_for (SEQ ID NO: 3) and non-ScGLC3_rv (SEQ ID NO: 4) were designed to amplify the full-length of the gene. PCR was carried out using chromosomal DNA of a genome sequencing strain, Saccharomyces pastorianus Weihenstephan 34/70 (sometimes abbreviated as "W34/70 strain"), as a template to obtain DNA fragments including the full-length gene of non-ScGLC3.

The non-ScGLC3 gene fragments thus obtained were inserted into pCR2.1-TOPO vector (Invitrogen) by TA cloning. The nucleotide sequences of the non-ScGLC3 gene were analyzed by Sanger's method (F. Sanger, Science, 214: 1215, 1981) to confirm the nucleotide sequence.

### Example 2: Analysis of Expression of non-ScGLC3 Gene during Beer Fermentation

A beer fermentation test was conducted using a lager brewing yeast, Saccharomyces pastorianus W34/70, and mRNA extracted from the lager brewing yeast during fermentation was detected by a beer yeast DNA microarray.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12.8×10⁶ cells/mL |

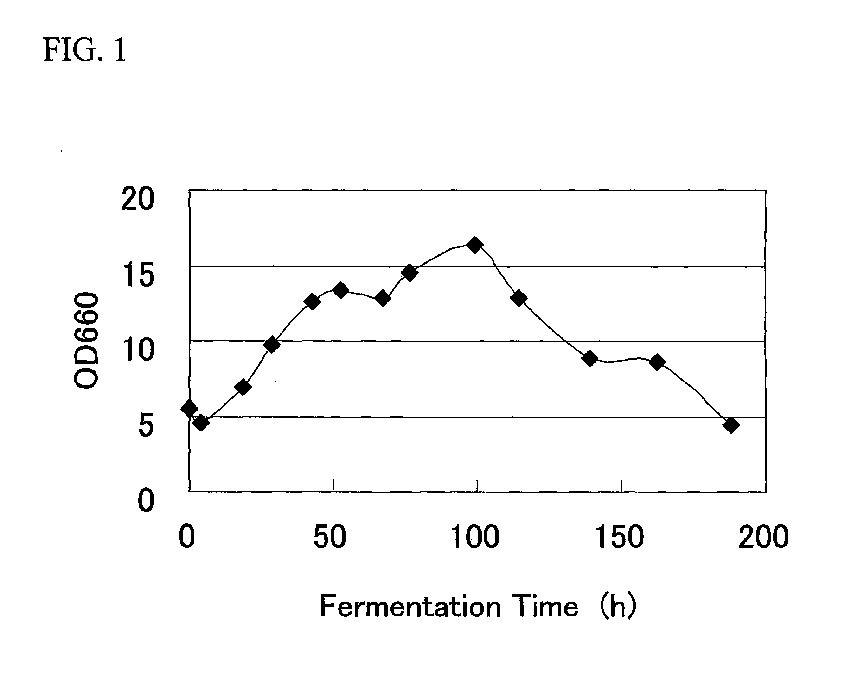

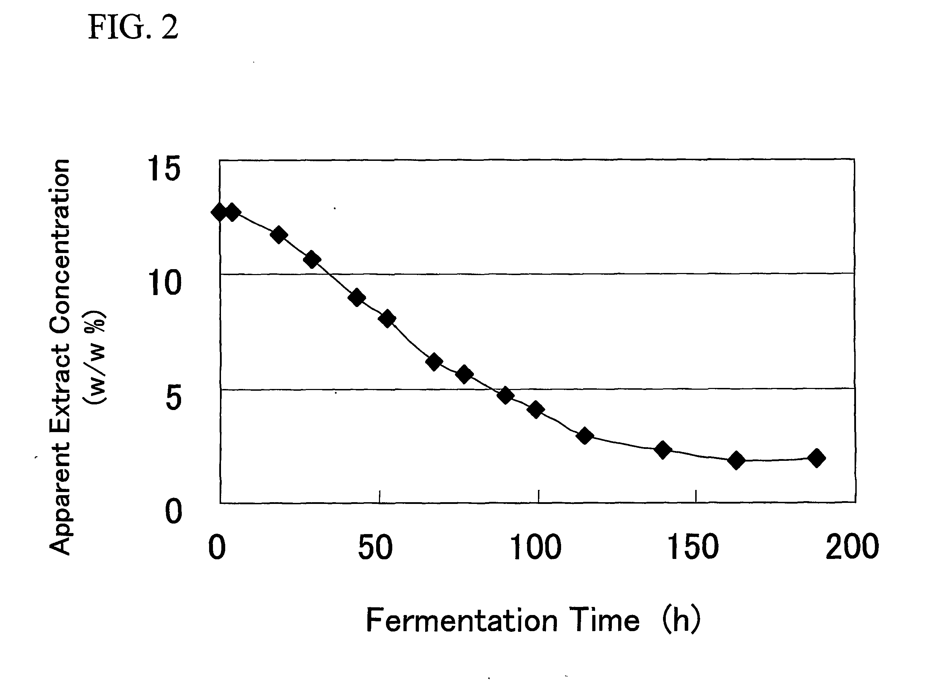

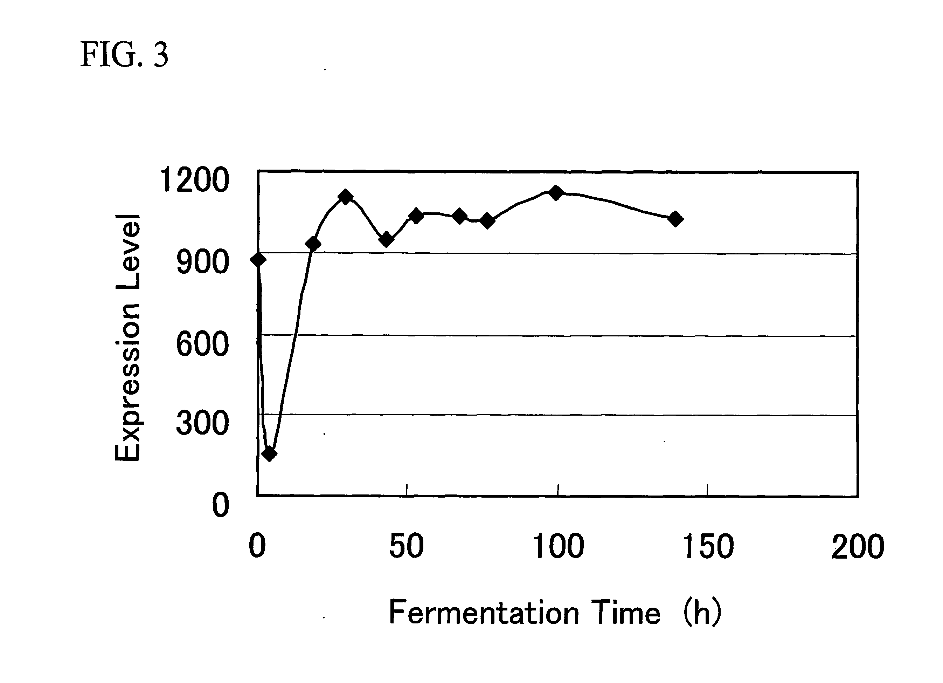

The fermentation liquor was sampled over time, and the time-course changes in amount of yeast cell growth (Fig. 1) and apparent extract concentration (Fig. 2) were observed. Simultaneously, yeast cells were sampled to prepare mRNA, and the prepared mRNA was labeled with biotin and was hybridized to a beer yeast DNA microarray. The signal was detected using GeneChip Operating system (GCOS; GeneChip Operating Software 1.0, manufactured by Affymetrix Co). Expression pattern of the non-ScGLC3 gene is shown in Figure 3. This result confirmed the expression of the non-ScGLC3 gene in the general beer fermentation.

### Example 3: Construction of non-ScGLC3 Highly Expressed Strain

The non-ScGLC3/pCR2.1-TOPO described in Example 1 was digested with the restriction enzymes SacI and NotI to prepare a DNA fragment containing the entire length of the protein-encoding region. This fragment was ligated to pYCGPYNot treated with the restriction enzymes SacI and NotI, thereby constructing the non-ScGLC3 high expression vector non-ScGLC3/pYCGPYNot. pYCGPYNot is a YCp-type yeast expression vector. A gene inserted is highly expressed by the pyruvate kinase gene PYK1 promoter. The geneticin-resistant gene G418^{r} is included as the selectable marker in the yeast, and the ampicillin-resistant gene Amp^{r} as the selectable marker in Escherichia coli.

Using the high expression vector prepared by the above method, an AJL4004 strain was transformed by the method described in Japanese Patent Application Laid-open No. H07-303475. The transformants were selected on a YPD plate medium (1% yeast extract, 2% polypeptone, 2% glucose and 2% agar) containing 300 mg/L of geneticin.

### Example 4: Evaluation of Drying-resistant Properly of non-ScGLC3 Highly Expressed Strain

Drying-resistant properties of the parent strain (AJL4004 strain) and the non-ScGLC3 highly expressed strain obtained by the method described in Example 3 were evaluated by a method described below.

One platinum loopful of each yeast was inoculated into 10 mL of wort containing 100 mg/L of geneticin, and stirred at 30°C overnight (precultivation). The precultivation liquid was inoculated into 10 mL wort containing 100 mg/L of geneticin to make its OD660 = 0.5, then main culture was initiated. The culture was continued for 2 days until the growth of the yeast reached stationary phase. Turbidity of the culture was measured at the completion of the culture, then the culture liquid was suspended in sterile water to make its OD = 2. One hundred microliter (100 µL) of the suspension thus obtained was dispensed into a 1.5 mL microtube, then the yeast cells were dried by evaporation for 1 hour using a reduced-pressure concentrator (DNA110 SpeedVac (registered trademark), manufactured by ThermoSavant).

Viable cell ratio was measured by a method described below. The dried yeast cells obtained above were resuspended in 50 µL of sterile water, then 50 µL of 0.02% methylene blue solution (pH 4.5) was added to the suspension. Blue-stained yeast cells which had lost reducing power were considered as dead yeast cells. Then the suspension was observed under a microscope, and viable cell ratio was measured using a Cell Vital Analyzer System (DA cell counter, manufactured by Yamato Scientific Co., Ltd.). The cells were counted until the population reached more than 2000 cells to minimize experimental error.

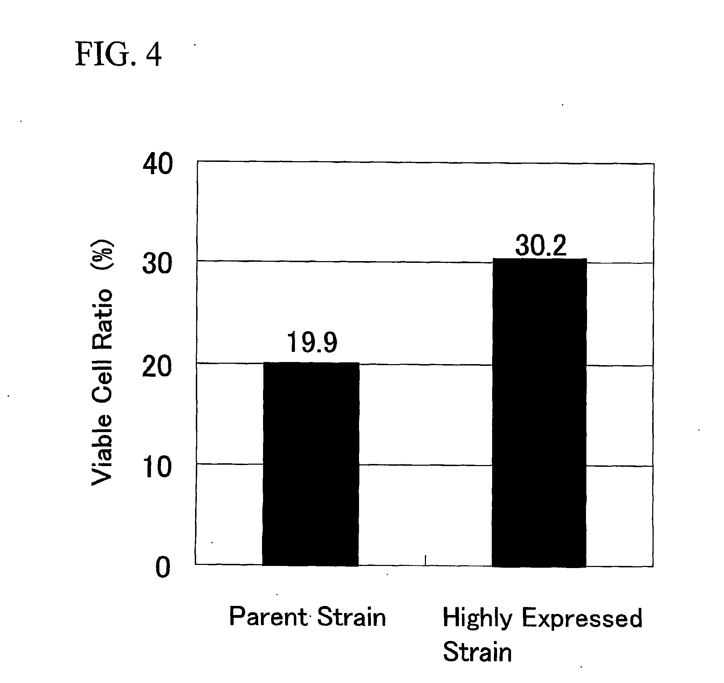

As indicated in Figure 4, viable cell ratio of the highly-expressed strain was 30.2%, though viable cell ratio of the parent strain was 19.9%. It was demonstrated by the results that drying-resistant property of yeast was increased by high expression of non-ScGLC3.

### Example 5: Evaluation of Low-temperature Resistant Property of non-ScGLC3 Highly Expressed Strain

Low-temperature resistant property of the parent strain (AJL4004 strain) and the non-ScGLC3 highly expressed strain obtained by the method described in Example 3 are evaluated by the method described below. Nine hundred microliter (900 µL) of the yeast suspensions cultured by the method described in Example 4 and prepared as OD660=2 are dispensed into two microtubes, respectively. One hundred microliter (100 µL) of sterile water is added to one of the microtubes, on the other hand, 100 µL of 99.5 % ethanol is added to another one (final concentration is 10%). The suspensions are stored at 5°C for 4 weeks, then viable cell ratios are measured by the same method as Example 4.

### INDUSTRIAL APPLICABILITY

According to the present invention, yeast can be stored stably for extended period of time, because drying-resistant property and/or low-temperature storage-resistant property can be enhanced by the present invention. Accordingly, efficiency of brewing alcoholic beverages (such as beer), production of bread, or manufacturing useful materials such as industrial alcohol production or production of useful proteins, etc., can be improved by the present invention.

## Claims

1. A polynucleotide selected from the group consisting of
(a) a polynucleotide comprising a polynucleotide consisting of the nucleotide sequence of SEQ ID NO: 1;
(b) a polynucleotide comprising a polynucleotide encoding a protein consisting of the amino acid sequence of SEQ ID NO: 2;
(c) a polynucleotide comprising a polynucleotide encoding a protein consisting of the amino acid sequence of SEQ ID NO: 2 in which 1 to 40 amino acids thereof are deleted, substituted, inserted and/or added, and having a glycogen branching enzyme activity; and
(d) a polynucleotide comprising a polynucleotide encoding a protein having an amino acid sequence having 94% or higher identity with the amino acid sequence of SEQ ID NO: 2, and said protein having a glycogen branching enzyme activity.

2. The polynucleotide according to Claim 1 selected from the group consisting of
(g) a polynucleotide comprising a polynucleotide encoding a protein consisting of the amino acid sequence of SEQ ID NO: 2, or encoding the amino acid sequence of SEQ ID NO: 2 in which 1 to 5 amino acids thereof are deleted, substituted, inserted, and/or added, and wherein said protein has a glycogen branching enzyme activity; and
(h) a polynucleotide comprising a polynucleotide encoding a protein having 99% or higher identity with the amino acid sequence of SEQ ID NO: 2, and having a glycogen branching enzyme activity.

3. The polynucleotide according to Claim 1 comprising a polynucleotide consisting of the nucleotide sequence of SEQ ID NO: 1.

4. The polynucleotide according to Claim 1 comprising a polynucleotide encoding a protein consisting of the amino acid sequence of SEQ ID NO: 2.

5. The polynucleotide according to any one of Claims 1 to 4, wherein the polynucleotide is DNA.

6. A protein encoded by the polynucleotide according to any one of Claims 1 to 5.

7. A vector containing the polynucleotide according to any one of Claims 1 to 5.

8. A yeast into which the vector according to Claim 7 has been introduced.

9. A method for producing an alcoholic beverage by using the yeast according to Claim 8.

10. The method according to Claim 9, wherein the brewed alcoholic beverage is a malt beverage.

11. The method according to Claim 9, wherein the brewed alcoholic beverage is wine.

12. Use of a polynucleotide of any one of claims 1 to 5 or of a protein of claim 6 or of a vector of claim 7 for increasing the drying-resistance in yeast cell.

13. A method for increasing the drying-resistance in a yeast cell comprising the step of transforming a yeast cell with a polynucleotide of any one of claims 1 to 5 or with a vector of claim 7 and expressing the glycogen branching enzyme encoded by the polynucleotide of any one of claims 1 to 5 in the transformed yeast cell.

## Patentansprüche

1. Polynucleotid ausgewählt aus der Gruppe bestehend aus:
(a) einem Polynucleotid, das ein Polynucleotid umfasst, das aus der Nucleotidsequenz von SEQ ID NO:1 besteht;
(b) einem Polynucleotid, das ein Polynucleotid umfasst, das ein Protein bestehend aus der Aminosäuresequenz von SEQ ID NO:2 codiert;
(c) einem Polynucleotid, das ein Polynucleotid umfasst, das ein Protein codiert, das aus der Aminosäuresquenz von SEQ ID NO:2 besteht, in der 1 bis 40 Aminosäuren davon deletiert, substituiert, insertiert und/oder hinzugefügt sind, und das Aktivität eines Glycogen-verzweigenden Enzyms hat; und
(d) einem Polynucleotid, das ein Polynucleotid umfasst, das ein Protein codiert, das eine Aminosäuresequenz hat, die 94% oder höhere Identität mit der Aminosäuresequenz von SEQ ID NO:2 hat, und wobei das Protein Aktivität eines Glycogenverzweigenden Enzyms hat.

2. Polynucleotid nach Anspruch 1, ausgewählt aus der Gruppe bestehend aus:
(g) einem Polynucleotid, das ein Polynucleotid umfasst, das ein Protein bestehend aus der Aminosäuresequenz von SEQ ID NO:2 codiert, oder die Aminosäuresequenz von SEQ ID NO:2 codiert, in der 1 bis 5 Aminosäuren davon deletiert, substituiert, insertiert und/oder hinzugefügt sind, und wobei das Protein Aktivität eines Glycogen-verzweigenden Enzyms hat; und
(h) einem Polynucleotid, das ein Polynucleotid umfasst, das ein Protein codiert, das 99% oder höhere Identität mit der Aminosäuresequenz von SEQ ID NO:2 hat und Aktivität eines Glycogen-verzweigenden Enzyms hat.

3. Polynucleotid nach Anspruch 1, das ein Polynucleotid umfasst, das aus der Nucleotidsequenz von SEQ ID NO:1 besteht.

4. Polynucleotid nach Anspruch 1, das ein Polynucleotid umfasst, das ein Protein bestehend aus der Aminosäuresequenz von SEQ ID NO:2 codiert.

5. Polynucleotid nach einem der Ansprüche 1 bis 4, wobei das Polynucleotid DNA ist.

6. Protein, das von dem Polynucleotid nach einem der Ansprüche 1 bis 5 codiert ist.

7. Vektor, der das Polynucleotid nach einem der Ansprüche 1 bis 5 umfasst.

8. Hefe, in die der Vektor nach Anspruch 7 eingeführt wurde.

9. Verfahren zur Herstellung eines alkoholischen Getränks unter Verwendung der Hefe nach Anspruch 8.

10. Verfahren nach Anspruch 9, wobei das gebraute alkoholische Getränk ein Malzgetränk ist.

11. Verfahren nach Anspruch 9, wobei das gebraute alkoholische Getränk Wein ist.

12. Verwendung eines Polynucleotids nach einem der Ansprüche 1 bis 5 oder eines Proteins nach Anspruch 6 oder eines Vektors nach Anspruch 7 zur Erhöhung der Trockenresistenz in einer Hefezelle.

13. Verfahren zur Erhöhung der Trockenresistenz in einer Hefezelle, das den Schritt des Transformierens einer Hefezelle mit einem Polynucleotid nach einem der Ansprüche 1 bis 5 oder mit einem Vektor nach Anspruch 7 umfasst und des Exprimierens des Glycogen-verzweigenden Enzyms in der transformierten Hefezelle, wobei das Enzym von dem Polynucleotid nach einem der Ansprüche 1 bis 5 codiert ist.

## Revendications

1. Polynucléotide choisi dans le groupe constitué par :
(a) un polynucléotide comprenant un polynucléotide constitué par la séquence de nucléotides de SEQ ID NO : 1 ;
(b) un polynucléotide comprenant un polynucléotide codant pour une protéine constituée par la séquence en acides aminés de SEQ ID NO : 2 ;
(c) un polynucléotide comprenant un polynucléotide codant pour une protéine constituée par la séquence en acides aminés de SEQ ID NO : 2 dans laquelle 1 à 40 des acides aminés de celle-ci sont délétés, substitués, insérés et/ou ajoutés, et ayant une activité d'enzyme de ramification du glycogène ; et
(d) un polynucléotide comprenant un polynucléotide codant pour une protéine ayant une séquence en acides aminés ayant 94 % ou plus d'identité avec la séquence en acides aminés de SEQ ID NO : 2, et ladite protéine ayant une activité d'enzyme de ramification du glycogène.

2. Polynucléotide selon la revendication 1 choisi dans le groupe constitué par :
(g) un polynucléotide comprenant un polynucléotide codant pour une protéine constituée par la séquence en acides aminés de SEQ ID NO : 2, ou codant pour la séquence en acides aminés de SEQ ID NO : 2 dans laquelle 1 à 5 des acides aminés de celle-ci sont délétés, substitués, insérés et/ou ajoutés, et dans laquelle ladite protéine a une activité d'enzyme de ramification du glycogène ; et
(h) un polynucléotide comprenant un polynucléotide codant pour une protéine ayant 99 % ou plus d'identité avec la séquence d'acides aminés de SEQ ID NO : 2, et ayant une activité d'enzyme de ramification du glycogène.

3. Polynucléotide selon la revendication 1 comprenant un polynucléotide constitué par la séquence de nucléotides de SEQ ID NO : 1.

4. Polynucléotide selon la revendication 1 comprenant un polynucléotide codant pour une protéine constituée par la séquence d'acides aminés de SEQ ID NO : 2.

5. Polynucléotide selon l'une quelconque des revendications 1 à 4, dans lequel le polynucléotide est de l'ADN.

6. Protéine codée par le polynucléotide selon l'une quelconque des revendications 1 à 5.

7. Vecteur contenant le polynucléotide selon l'une quelconque des revendications 1 à 5.

8. Levure dans laquelle le vecteur selon la revendication 7 a été introduit.

9. Procédé de production d'une boisson alcoolisée utilisant la levure selon la revendication 8.

10. Procédé selon la revendication 9, dans laquelle la boisson alcoolisée fermentée est une boisson à base de malt.

11. Procédé selon la revendication 9, dans laquelle la boisson alcoolisée fermentée est le vin.

12. Utilisation d'un polynucléotide selon l'une quelconque des revendications 1 à 5 ou d'une protéine selon la revendication 6 ou d'un vecteur selon la revendication 7 pour accroître la résistance à la sécheresse chez une cellule de levure.

13. Procédé pour accroître la résistance à la sécheresse chez une cellule de levure comprenant l'étape de transformation d'une cellule de levure avec un polynucléotide selon l'une quelconque des revendications 1 à 5 ou avec un vecteur selon la revendication 7 et exprimant l'enzyme de ramification du glycogène codée par le polynucléotide selon l'une quelconque des revendications 1 à 5 chez la cellule de levure transformée.
